# EUROPÄISCHE PATENTSCHRIFT

(11) **EP 1 480 960 B1**
(45) Veröffentlichungstag und Bekanntmachung des Hinweises auf die Patenterteilung: **09.05.2012**
(21) Anmeldenummer: 03742936.2
(22) Anmeldetag: 14.02.2003
(51) Int. Cl.: C07D 271/113, A61K 31/4245, A61P 3/04

(54) **Substituierte 3-Phenyl-5-alkoxy-1,3,4-oxadiazol-2-one sowie ihre Herstellung und Verwendung in Arzneistoffen**
Substituted 3-phenyl-5-alkoxy-1,3,4-oxadiazol-2-ones, the production thereof and their use in medicaments
3-Phényl-5-alcoxy-1,3,4-oxdiazol-2-ones substitués, leur production et leur utilisation aux médicaments

(30) Priorität: 28.02.2002 DE 10208987
(43) Veröffentlichungstag der Anmeldung: 01.12.2004
(73) Patentinhaber: Sanofi-Aventis Deutschland GmbH, 65929 Frankfurt am Main (DE)
(72) Erfinder: SCHOENAFINGER, Karl, 63755 Alzenau (DE); PETRY, Stefan, 65779 Kelkheim (DE); MÜLLER, Günter, 65843 Sulzbach a. Ts. (DE); BAUER, Armin, 65843 Sulzbach (DE); HEUER, Hubert, Otto, 55270 Schwabenheim (DE)
(86) Internationale Anmeldenummer: PCT/EP2003/001484
(87) Internationale Veröffentlichungsnummer: WO 2003/072555

(56) Entgegenhaltungen:
- WO-A-01/17981
- WO-A-01/66531

## Beschreibung

Die Erfindung betrifft substituierte 3-Phenyl-5-alkoxy-1,3,4-oxadiazol-2-one, die eine hemmende Wirkung an der pankreatischen Lipase, PL, zeigen.

Bestimmte 5-Alkoxy-1,3,4-oxadiazol-2-one mit einem ortho-substituierten Phenylring als Substituenten oder mit ankondensierten fünf- oder sechsgliedrigen Ringen besitzen anthelmintische (DE-A 26 04 110) und insektizide Wirkung (DE-A 26 03 877, EP-B 0 048 040, EP-B 0 067 471).

Bestimmte 5-Phenoxy-1,3,4-oxadiazol-2-one mit einem ortho-substituierten Phenylring als Substituenten zeigen endoparasitizide Wirkung (EP-A 0 419 918).

Substituierte 3-Phenyl-5-alkoxy-1,3,4-oxadiazol-2-one mit einer hemmenden Wirkung an der hormonsensitiven Lipase sind aus WO 01/17981 (HMR 1999/L 052) und WO 01/66531 (AVE-D 2000/A 015K) bekannt.

Ziel der Erfindung war es nun, Verbindungen zu finden, die eine hemmende Wirkung an der pankreatischen Lipase, PL, zeigen.

Dies wurde erreicht mit den substituierten 3-Phenyl-5-alkoxy-1,3,4-oxadiazol-2-onen der allgemeinen Formel 1, 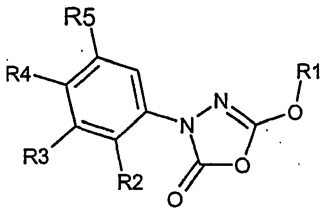 worin bedeuten:
- R¹: C₇-C₂₂-Alkyl oder C₇-C₂₀-Alkenyl;
- R², R³, R⁴ und R⁵: unabhängig voneinander Wasserstoff, Halogen, Nitro, , C₁-C₄-Alkyl, C₁-C₉-Alkyloxy, Trifluormethyl, Trifluormethoxi, oder C₆-C₁₀-Aryl-C₁-C₄-alkyloxy, C₆-C₁₀-Aryloxy, C₆-C₁₀-Aryl, C₃-C₈-Cycloalkyl oder O-C₃-C₈-Cycloalkyl, die durch Halogen, Trifluormethyl, C₁-C₄-Alkyloxy oder C₁-C₄-Alkyl einfach, doppelt oder dreifach substituiert sein können; sowie deren pharmakologisch verträglichen Salze und Säureadditionssalze.

Halogen steht für Fluor, Chlor, Brom, bevorzugt für Fluor und Chlor. Alkyl, Alkenyl, Alkoxy usw. können verzweigt oder unverzweigt sein.

Bevorzugt sind ferner Verbindungen der Formel 1, worin bedeuten:
R² Wasserstoff, Halogen, C₁-C₄-Alkyl oder C₁-C₉-Alkyloxy;
   und/oder
R³ Wasserstoff, C₁-C₄-Alkyl, Trifluormethoxi, C₆-C₁₀-Aryl-C₁-C₄-alkyloxy, das
   gegebenenfalls im Arylteil durch Halogen substituiert sein kann;
   und/oder
R⁴ Wasserstoff, Trifluormethoxy oder Chlorphenoxy;
   und/oder
   R⁵ Wasserstoff.

Besonders bevorzugt sind Verbindungen der Formel 1, worin R¹ C₈-C₁₆-Alkyl bedeutet.

Ganz besonders bevorzugt sind Verbindungen der Formel 1 worin bedeuten:
- R¹: C₈-C₁₆-Alkyl,
- R²: Wasserstoff,
- R³: Wasserstoff oder Trifluormethyloxy,
- R⁴: Wasserstoff, Trifluormethyloxy oder 4-Chlor-phenoxy und
- R⁵: Wasserstoff.

Ganz besonders bevorzugt sind ferner die in den Beispielen 1, 2, 3, 4, 5, 6 und 16 genannten Verbindungen der Formel 1.

Die erfindungsgemäßen Verbindungen der allgemeinen Formel 1 besitzen eine hemmende Wirkung an der pankreatischen Lipase, PL.

Die Erfindung bezieht sich die Verbindungen der Formel 1, in Form ihrer Racemate, racemischen Mischungen und reinen Enantiomere sowie auf ihre Diastereomere und Mischungen davon.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der Verbindungen der Formel 1 sind Salze anorganischer Säuren, wie Salzsäure, Bromwasserstoff-, Phosphor-, Metaphosphor-, Salpeter-, Sulfam- und Schwefelsäure sowie organischer Säuren, wie z.B. Essigsäure, Benzolsulfon-, Benzoe-, Zitronen-, Ethansulfon-, Fumar-, Glucon-, Glykol-, Isäthion-, Milch-, Lactobion-, Malein-, Apfel-, Methansulfon-, Bernstein-, p-Toluolsulfon-, Wein- und Trifluoressigsäure. Für medizinische Zwecke wird in besonders bevorzugter Weise das Chloridsalz und das Weinsäuresalz, verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nichttherapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung, z.B. ein Ester, das bei Verabreichung an einen Säuger, wie z.B. den Menschen, in der Lage ist, (direkt oder indirekt) eine solche Verbindung oder einen aktiven Metaboliten hiervon zu bilden.

Ein weiterer Aspekt dieser Erfindung ist die Verwendung von Prodrugs der Verbindungen der Formel 1. Solche Prodrugs können in vivo zu einer Verbindung der Formel 1 metabolisiert werden. Diese Prodrugs können selbst wirksam sein oder nicht.

Die Verbindungen der Formel 1 können auch in verschiedenen polymorphen Formen vorliegen, z.B. als amorphe und kristalline polymorphe Formen. Alle polymorphen Formen der Verbindungen der Formel 1 gehören in den Rahmen der Erfindung und sind ein weiterer Aspekt der Erfindung.

Nachfolgend beziehen sich alle Verweise auf "Verbindung(en) gemäß Formel 1" auf Verbindung(en) der Formel 1 wie vorstehend beschrieben, sowie ihre Salze, Solvate und physiologisch funktionellen Derivate wie hierin beschrieben.

Die Menge einer Verbindung gemäß Formel 1, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht des Salzes der Verbindung der Formel 1. Zur Prophylaxe oder Therapie der oben genannten Zustände können die Verbindungen gemäß Formel 1 selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel 1. Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel 1 abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel 1 enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel 1 mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel 1, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel 1 mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Die Herstellung der erfindungsgemäßen Verbindungen der allgemeinen Formel 1 kann nach an und für sich bekannten Methoden auf verschiedenen Wegen erfolgen.

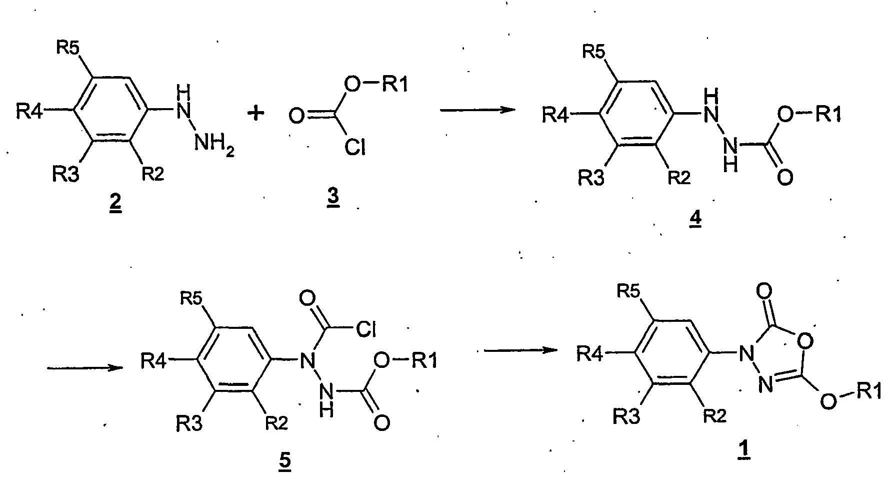

Beispielsweise kann die Herstellung der substituierten 3-Phenyl-5-alkoxy-1,3,4-oxadiazol-2-one der allgemeinen Formel 1 erfolgen, durch Umsetzung von Hydrazinen der allgemeinen Formel 2 mit Chlorameisensäureestern der Formel 3 oder anderen reaktiven Kohlensäureesterderivaten, worin R¹, R², R³, R⁴ und R⁵ wie oben definiert sind, zu den Verbindungen der Formel 4, welche mit Phosgen, Carbonyldiimidazol, Diphosgen oder Triphosgen acyliert, zyklisiert und gegebenenfalls durch weitere chemische Modifikation der Reste R²-R⁵, wie beispielsweise durch Reduktion von Nitro- zu Aminoresten nach bekannten Verfahren, und anschließende Acylierung oder Alkylierung, zu den Verbindungen der Formel 1 umgesetzt werden. Da bei diesen Reaktionen in der Regel Säuren freigesetzt werden, empfiehlt es sich zur Beschleunigung Basen wie Pyridin, Triethylamin, Natronlauge oder Alkalicarbonate zuzusetzen. Die Reaktionen können in weiten Temperaturbereichen durchgeführt werden. In der Regel hat es sich als vorteilhaft herausgestellt, bei 0°C bis zum Siedepunkt des verwendeten Lösungsmittels zu arbeiten. Als Lösemittel kommen beispielsweise Methylenchlorid, THF, DMF, Toluol, Essigester, nHeptan, Dioxan, Diethylether zu Einsatz.

Die Hydrazine der Formel 2 lassen sich nach bekannten Methoden z.B. durch Diazotierung der entsprechenden Aniline und 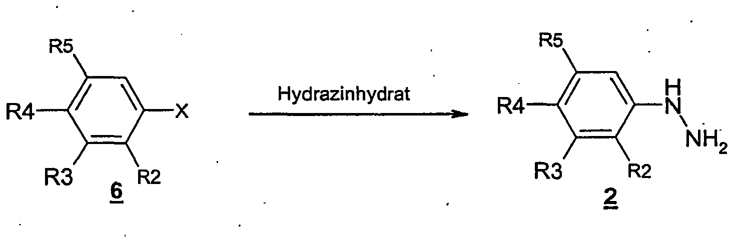 nachfolgender Reduktion nach bekannten Methoden oder durch nukleophile Substitution von geeignet substituierten Phenylderivaten 6 (X= F, Cl, Br, I, OSO₂CF₃) mit Hydrazinhydrat herstellen. Solche geeignete Phenylderivate können Nitro substituierte Halogenbenzole, vorzugsweise Fluor- und Chlor-nitrobenzole sein, aus denen sich an geeigneter Stelle im Syntheseweg durch Reduktion und Umsetzung mit Acylierungs- oder Alkylierungsmitteln ,wie beispielweise Säurechloriden, Anhydriden, Isocyanaten, Chlorameisensäureestern ,Sulfonsäurechloriden oder Alkyl- und Arylalkyl-halogeniden, oder durch reduktive Alkylierung mit Aldehyden nach bekannten Methoden die erfindungsgemäßen Verbindungen herstellen lassen.

Die Wirkung der erfindungsgemäßen Verbindungen der Formel 1 wurde an folgendem Enzymtestsytem geprüft:

Die Verbindungen der Formel 1 zeigen eine hemmende Wirkung an der pankreatischen Lipase (PL). Als Inhibitoren der PL können sie die Resorption des mit der Nahrung aufgenommenen Fettes verhindern und dadurch zu einer Reduktion der Fettaufnahme und des Körpergewichtes führen oder eine Zunahme des Körpergewichtes verhindern. Die Verbindungen der Formel 1 sind besonders zur Behandlung von Obesitas geeignet, können aber auch bei verschiedenen Entgleisungen des Stoffwechsels, wie zum Beispiel Diabetes, des Herz-Kreislaufsystems, wie zu Beispiel Bluthochdruck und Herzinfarkt, einen sehr günstigen Einfluss ausüben.

Die Wirksamkeit der Verbindungen wurde wie folgt getestet:
1. Herstellung des Substrats:
   80 µl Tripalmitin (85 mM in Chloroform) werden mit 5 µl Glyzerintri[9,10(n)-³H]oleat (5 mCi/ml in Toluol) in einem 12-ml Polypropylengefäß gemischt. Nach Einrotieren (50°C) und Hinzugabe von 4 ml 200 mM Tris/HCl (pH 7.6), 0.8 % TX-1 00 wird die Mischung mit Ultraschall behandelt (Branson Sonifier B-12, Leistungsstufe 4, 3 mal 2 min mit 1-min Intervallen auf Eis) bis eine homogene milchig trübe Suspension entstanden ist.
2. Assay:
   Lipase Puffer: 80 mM Tris/HCl (pH 7.6), 600 mM NaCl, 8 mM CaCl₂, 8 mM benzamidine, 2 mM Pefabloc (Roche Biochemicals)(Inhibitoren erst am Testtag zugeben)

Pankreatische Lipase: Angereicherte Präparation aus Schweinpankreas (Sigma Bestell Nr. L-0382) gelöst in Lipase Puffer (100000 units/500 µl)

Durchführung:
5 µl Testsubstanz (in 100 % DMSO) oder DMSO (Kontrolle) werden mit 10 µl Substrat und 5 µl Lipase (in dieser Reihenfolge) gemischt und für 30 min bei 30°C inkubiert (Eppendorf Thermomixer, 350 min⁻¹). Nach Zugabe von 325 µl Methanol/Chloroform/nHeptan (10/9/7) und 105 µl 0.1 M K₂CO₃, 0.1 M H₃BO₃ (pH 10,5 mit 1 M KOH eingestellt) und kräftigem Mischen wird zur Phasentrennung zentrifugiert (8000 rpm, Eppendorfzentrifuge, 4°C). Je 140 µl des wässrigen Überstandes (enthält das freigesetzte radiomarkierte Oleat; 70 % recovery) werden in 20 ml Szintillationsgefäße überführt und mit 6 ml Szintillationscocktail (Beckman ReadySafe) versetzt. Nach kräftigem Mischung und Inkubation für 2 h bei Raumtemperatur erfolgt die Radioaktivitätsmessung im Flüssigszintillationszähler (Beckman, L8008, Tritium-Kanal mit Quenchkurve, 20 min Messzeit).
Auswertung:
   Substanzen werden in jeder Konzentration routinemäßig in drei unabhängigen Inkubations-Ansätzen jeweils mit Doppelbestimmung nach Phasentrennung geprüft (SD < 0.02). Von allen Werten werden Backgroundwerte (Reaktion unter gleichen Bedingungen aber ohne Lipase) abgezogen (entspricht überwiegend dem Anteil von Glyzerintrioleat bzw. freies Oleat in der Substratpräparation in der wässrigen Phase, < 5 % der eingesetzten Radioaktivität). Die Hemmung der enzymatischen Aktivität der pankreatischen Lipase durch eine Testsubstanz wird über den Vergleich mit einer nicht-gehemmten Kontrollreaktion bestimmt (Gegenwart von Lipase = 0 % Hemmung; Abwesenheit von Lipase 100 % Hemmung jeweils nach Backgroundkorrektur). Die Berechnung des IC₅₀-Wertes erfolgt über eine Hemmkurve mit bis zu 8 Konzentrationen der Testsubstanz. Zur Kurvenanpassung und IC₅₀-Ermittlung wird das Softwarepaket GRAPHIT (Elsevier-BIOSOFT) benutzt.

In diesem Test zeigten die Verbindungen die folgende Wirkung:

| Verbindung Beispiel Nr. | IC₅₀ (µM) |
|---|---|
| 1 | 0,03 |
| 2 | 0,25 |
| 3 | 0,35 |
| 4 | 2,5 |
| 5 | 2,0 |
| 6 | 0,9 |
| 15 | 0,6 |

Die nachfolgenden Beispiele erläutern die Herstellungsmethoden näher, ohne sie einzuschränken.

### Beispiele:

### Beispiel 1:

5-Dodecyloxy-3-(4-trifluormethoxy-phenyl)-3H-(1,3,4)-oxadiazol-2-on

Zur Mischung bestehend aus 0,84 g 4-Trifluormethoxy-phenylhydrazin, 15 ml NMP und 2 ml Pyridin wurden vorsichtig unter Eiskühlung 0,43 ml Chlorameisensäuredodecylester zugetropft und dann unter langsamen Erwärmen auf RT 2 Stunden gerührt. Nach Verdünnen mit 50 ml Wasser wurde mit 30 ml Methylenchlorid ausgeschüttelt, die organische Fase mit Natriumsulfat getrocknet und mit 5ml Pyridin und unter Umrühren und Eiskühlung mit 3 ml einer 20%igen toluolischen Phosgenlösung tropfenweise versetzt. Diese Mischung stand über Nacht bei Raumtemperatur und wurde mit weiteren 10 ml Methylenchlorid verdünnt und dann 3 mal mit Wasser gewaschen. Nach dem Trocknen über Natriumsulfat wurde die Mischung im Vakuum eingeengt und das Produkt durch Säulenchromatografie (Kieselgel, Lösungsmittel: Methanol:Methylenchlorid = 2 : 98) gereinigt. Ausbeute:0,85 g Fp.: 41°C

Analog wurden die Verbindungen der folgenden Beispiele hergestellt:

### Beispiel 2:

5-Hexadecyloxy-3-(4-trifluormethoxy-phenyl)-3H-(1,3,4)-oxadiazol-2-on
Fp.: 56°C

### Beispiel 3:

5-Octyloxy-3-(4-trifluormethoxy-phenyl)-3H-(1,3,4)-oxadiazol-2-on
Fp.: Öl

### Beispiel 4:

5-Hexadecyloxy-3-(3-trifluormethoxy-phenyl)-3H-(1,3,4)-oxadiazol-2-on
Fp.: 53°C

### Beispiel 5:

5-Hexadecyloxy-3-(4-(4-chlorphenoxy)-phenyl)-3H-(1,3,4)-oxadiazol-2-on
Fp.: 52°C

### Beispiel 6:

5-Octyloxy-3-phenyl-3H-(1,3,4)-oxadiazol-2-on
Fp.: 38°C

### Beispiel 7:

5-Octyloxy-3-(3-fluor-phenyl)-3H-(1,3,4)-oxadiazol-2-on
Fp.: Öl

### Beispiel 8:

5-Hexadecyloxy-3-(3-fluor-phenyl)-3H-(1,3,4)-oxadiazol-2-on
Fp.: 58°C

### Beispiel 9:

5-Hexadecyloxy-3-(3-benzyloxy-phenyl)-3H-(1,3,4)-oxadiazol-2-on
Fp.: 65°C

### Beispiel 10:

5-Hexadecyloxy-3-phenyl-3H-(1,3,4)-oxadiazol-2-on
Fp.: 63°C

### Beispiel 11:

5-Hexadecyloxy-3-(4-nitro-phenyl)-3H-(1,3,4)-oxadiazol-2-on
Fp.: 72°C

### Beispiel 12:

5-Hexadecyloxy-3-(4-methoxy-phenyl)-3H-(1,3,4)-oxadiazol-2-on
Fp.: 66°C

### Beispiel 13:

5-Hexadecyloxy-3-(4-benzyloxy-phenyl)-3H-(1,3,4)-oxadiazol-2-on
Fp.: 73°C

### Beispiel 14:

5-Decyloxy-3-(4-trifluormethoxy-phenyl)-3H-(1,3,4)-oxadiazol-2-on
Fp.: Öl

### Beispiel 15:

5-Undecyloxy-3-(4-trifluormethoxy-phenyl)-3H-(1,3,4)-oxadiazol-2-on
Fp.: 38°C

### Beispiel 16:

5-Tetradecyloxy-3-(4-trifluormethoxy-phenyl)-3H-(1,3,4)-oxadiazol-2-on
Fp.: 46°C

### Beispiel 17:

5-Tridecyloxy-3-(4-trifluormethoxy-phenyl)-3H-(1,3,4)-oxadiazol-2-on
Fp.: 50°C

### Beispiel 18:

5-(2-(2-Hexyloxy-ethoxy)-ethoxy)-3-(4-trifluormethoxy-phenyl)-3H-(1,3,4)-oxadiazol-2-on
Fp.: Öl

### Beispiel 19:

5-((Z)-Octadec-9-enyloxy)-3-(4-trifluormethoxy-phenyl)-3H-(1,3,4)-oxadiazol-2-on
Fp.: Öl

### Beispiel 20:

5-(Dodecyloxy-ethoxy)-3-(4-trifluormethoxy-phenyl)-3H-(1,3,4)-oxadiazol-2-on
Fp.: Öl

### Beispiel 21:

5-(2-(4-Fluorphenyl)-ethoxy)-3-(4-trifluormethoxy-phenyl)-3H-(1,3,4)-oxadiazol-2-on
Fp.: 60°C

### Beispiel 22:

5-((3β-Cholestan-3-yl)-oxy)--3-(4-trifluormethoxy-phenyl)-3H-(1,3,4)-oxadiazol-2-on
Fp.: 127°C

### Beispiel 23:

5-(2-Butoxy-ethoxy)-3-(4-trifluormethoxy-phenyl)-3H-(1,3,4)-oxadiazol-2-on
Fp.: Harz

### Beispiel 24:

5-(7-Phenyl-heptyloxy)-3-(4-trifluormethoxy-phenyl)-3H-(1,3,4)-oxadiazol-2-on
Fp.: Harz

### Beispiel 25:

5-(Docosyloxy-ethoxy)-3-(4-trifluormethoxy-phenyl)-3H-(1,3,4)-oxadiazol-2-on
Fp.: 71 °C

### Beispiel 26:

5-(2-(1-Naphthloxy)-ethoxy)-3-(4-trifluormethoxy-phenyl)-3H-(1,3,4)-oxadiazol-2-on
Fp.: Harz

### Beispiel 27:

5-(4-Octylphenoxy)-3-(4-trifluormethoxy-phenyl)-3H-(1,3,4)-oxadiazol-2-on
Fp.: Harz

### Beispiel 28:

5-(3-Phenoxy-phenoxy)-3-(4-trifluormethoxy-phenyl)-3H-(1,3,4)-oxadiazol-2-on
Fp.: Harz

### Beispiel 29:

5-(Dodecyloxy)-3-(4-trifluormethoxy-phenyl)-3H-(1,3,4)-oxadiazol-2-on
Fp.: 41°C

### Beispiel 30:

5-(Dodecyloxy)-3-(3,4-dichlor-phenyl)-3H-(1,3,4)-oxadiazol-2-on
Fp.: 74°C

### Beispiel 31:

5-(Dodecyloxy)-3-(3,5-dichlor-phenyl)-3H-(1,3,4)-oxadiazol-2-on
Fp.: 48°C

### Beispiel 32:

5-(Dodecyloxy)-3-(3-methoxy-phenyl)-3H-(1,3,4)-oxadiazol-2-on
Fp.: 51°C

### Beispiel 33:

5-(Dodecyloxy)-3-(4-methoxy-phenyl)-3H-(1,3,4)-oxadiazol-2-on
Fp.: 57°C

### Beispiel 34:

5-(Dodecyloxy)-3-(3-nitro-phenyl)-3H-(1,3,4)-oxadiazol-2-on
Fp.: 64°C

### Beispiel 35:

5-(Dodecyloxy)-3-(3-trifluormethyl-phenyl)-3H-(1,3,4)-oxadiazol-2-on
Fp.: 43°C

### Beispiel 36:

5-(Dodecyloxy)-3-(3,5-bis-trifluormethyl-phenyl)-3H-(1,3,4)-oxadiazol-2-on
Fp.: Öl

### Beispiel 37:

5-(Dodecyloxy)-3-(4-benzyloxy-phenyl)-3H-(1,3,4)-oxadiazol-2-on
Fp.: 65°C

### Beispiel 38:

5-(Dodecyloxy)-3-(3-fluor-phenyl)-3H-(1,3,4)-oxadiazol-2-on
Fp.: 44°C

### Beispiel 39:

5-(Dodecyloxy)-3-(3-(4-fluorbenzyloxy)-4-nitro-phenyl)-3H-(1,3,4)-oxadiazol-2-on
Fp.: 71°C

### Beispiel 40:

5-(Dodecyloxy)-3-(2-methyl-4-nitro-phenyl)-3H-(1,3,4)-oxadiazol-2-on
Fp.: 63°C

### Beispiel 41:

5-(Dodecyloxy)-3-(3-methyl-4-nitro-phenyl)-3H-(1,3,4)-oxadiazol-2-on
Fp.: 62°C

## Patentansprüche

1. Verbindungen der allgemeinen Formel 1 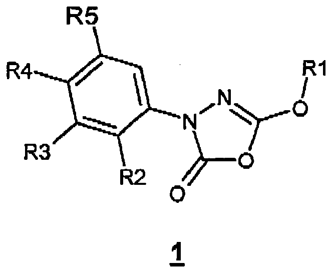 worin bedeuten:
R¹ C₇-C₂₂-Alkyl oder C₇-C₂₀-Alkenyl bedeutet;
R², R³, R⁴ und R⁵ unabhängig voneinander Wasserstoff, Halogen, Nitro, C₁-C₄-Alkyl, C₁-C₉-Alkyloxy, Trifluormethyl, Trifluormethoxi, oder C₆-C₁₀-Aryl-C₁-C₄-alkyloxy, C₆-C₁₀-Aryloxy, C₆-C₁₀-Aryl, C₃-C₈-Cycloalkyl oder O-C₃-C₈-Cycloalkyl, die durch Halogen, CF₃, C₁-C₄-Alkyloxy oder C₁-C₄-Alkyl einfach, doppelt oder dreifach substituiert sein können; sowie deren pharmakologisch verträglichen Salze und Säureadditionssalze.

2. Verbindungen der Formel 1 gemäß Anspruch 1, worin
R² Wasserstoff, Halogen, C₁-C₄-Alkyl oder C₁-C₉-Alkylox bedeutet.

3. Verbindungen der Formel 1 gemäß den Ansprüchen 1 bis 2, worin R³ Wasserstoff, C₁-C₄-Alkyl, Trifluormethoxi, C₆-C₁₀-Aryl-C₁-C₄-alkyloxy, das gegebenenfalls im Arylteil durch Halogen substituiert sein kann, bedeutet.

4. Verbindungen der Formel 1 gemäß den Ansprüchen 1 bis 3, worin
R⁴ Wasserstoff, Trifluormethoxi oder Chlorphenoxy bedeutet.

5. Verbindungen der Formel 1 gemäß den Ansprüchen 1 bis 4, worin
R⁵ Wasserstoff bedeutet.

6. Verbindungen der Formel 1 gemäß den Ansprüchen 1 bis 5, worin
R¹ C₈-C₁₆-Alkyl bedeutet.

7. Verbindungen der Formel 1 gemäß den Ansprüchen 1 bis 6, worin
R¹ C₈-C₁₆-Alkyl,
R² Wasserstoff,
R³ Wasserstoff oder Trifluormethyloxi,
R⁴ Wasserstoff, Trifluormethyloxi oder 4-Chlor-phenoxy und
R⁵ Wasserstoff bedeuten

8. Verfahren zur Herstellung der Verbindungen der Formel 1 gemäß den Ansprüchen 1 bis 7, **gekennzeichnet durch** 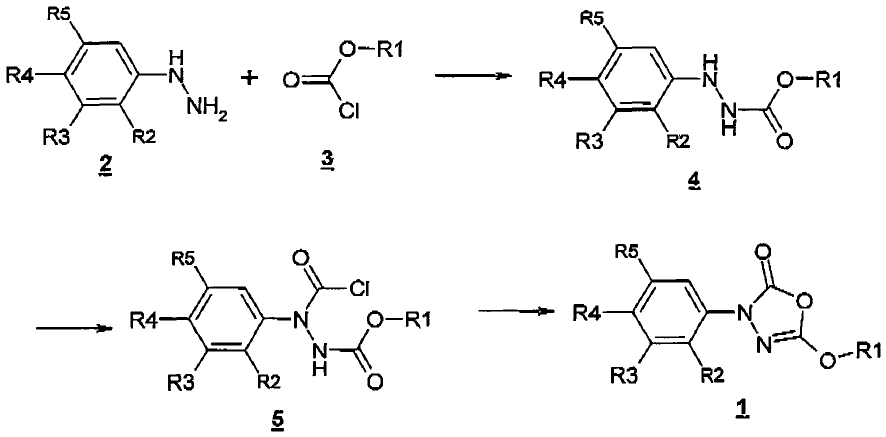 Umsetzung von Hydrazinen der allgemeinen Formel 2 mit Chlorameisensäureestern der Formel 3 oder anderen reaktiven Kohlensäureesterderivaten, worin R¹, R², R³, R⁴ und R⁵ wie in den Ansprüchen 1 bis 7 definiert sind, zu den Verbindungen der Formel 4, welche mit Phosgen, Carbonyldiimidazol, Diphosgen oder Triphosgen acyliert, zyklisiert und gegebenenfalls **durch** weitere chemische Modifikation der Reste R²-R⁵ zu den Verbindungen der Formel 1 umgesetzt werden.

9. Arzneimittel enthaltend mindestens eine Verbindung der Formel 1 gemäß den Ansprüchen 1 bis 7.

10. Arzneimittel zur Behandlung von Obesitas enthaltend mindestens eine Verbindung der Formel 1 gemäß den Ansprüchen 1 bis 7.

11. Verwendung mindestens einer der Verbindungen der Formel 1 gemäß den Ansprüchen 1 bis 7 zur Herstellung eines Heilmittels.

## Claims

1. Compounds of the formula 1 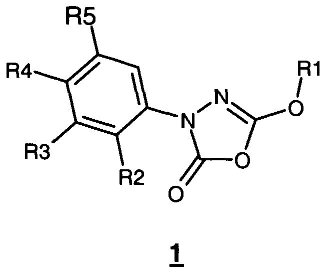 in which the meanings are:
R¹ C₇-C₂₂-alkyl or C₇-C₂₀-alkenyl;
R², R³, R⁴ and R⁵ independently of one another
hydrogen, halogen, nitro, C₁-C₄-alkyl, C₁-C₉-alkyloxy, trifluoromethyl, trifluoromethoxy, or
C₆-C₁₀-aryl-C₁-C₄-alkyloxy, C₆-C₁₀-aryloxy, C₆-C₁₀-aryl, C₃-C₈-cycloalkyl or O-C₃-C₈-cycloalkyl, each of which may be substituted once, twice or three times by halogen, CF₃, C₁-C₄-alkyloxy or C₁-C₄-alkyl;
and their pharmacologically acceptable salts and acid addition salts.

2. Compounds of the formula 1 as claimed in claim 1, in which
R² is hydrogen, halogen, C₁-C₄-alkyl or C₁-C₉-alkyloxy.

3. Compounds of the formula 1 as claimed in claims 1 to 2, in which
R³ is hydrogen, C₁-C₄-alkyl, trifluoromethoxy, C₆-C₁₀-aryl-C₁-C₄-alkyloxy which may optionally be substituted in the aryl moiety by halogen.

4. Compounds of the formula 1 as claimed in claims 1 to 3, in which
R⁴ is hydrogen, trifluoromethoxy or chlorophenoxy.

5. Compounds of the formula 1 as claimed in claims 1 to 4, in which
R⁵ is hydrogen.

6. Compounds of the formula 1 as claimed in claims 1 to 5, in which
R¹ is C₈-C₁₆-alkyl.

7. Compounds of the formula 1 as claimed in claims 1 to 6, in which
R¹ is C₈-C₁₆-alkyl,
R² is hydrogen,
R³ is hydrogen or trifluoromethyloxy,
R⁴ is hydrogen, trifluoromethyloxy or 4-chlorophenoxy and
R⁵ is hydrogen.

8. A process for preparing the compounds of the formula 1 as claimed in claims 1 to 7, which comprises 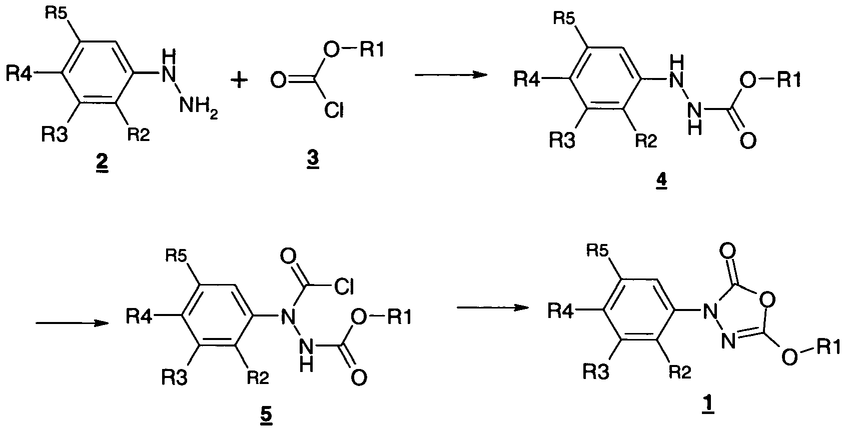 reacting hydrazines of the formula 2 with chloroformic esters of the formula 3 or other reactive carbonic ester derivatives, in which R¹, R², R³, R⁴ and R⁵ are as defined in claims 1 to 7, to give the compounds of the formula 4, which are acylated with phosgene, carbonyldiimidazole, diphosgene or triphosgene, cyclized and converted where appropriate by further chemical modification of the radicals R²-R⁵ into the compounds of the formula 1.

9. A medicament comprising at least one compound of the formula 1 as claimed in claims 1 to 7.

10. A medicament for the treatment of obesity comprising at least one compound of the formula 1 as claimed in claims 1 to 7.

11. The use of at least one of the compounds of the formula 1 as claimed in claims 1 to 7 for producing a medicine.

## Revendications

1. Composés de formule générale 1 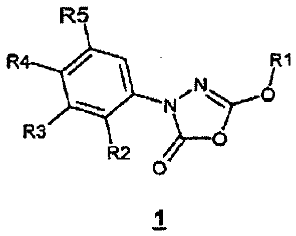 dans laquelle :
R¹ représente un groupe alkyle en C₇-C₂₂ ou alcényle en C₇-C₂₀ ;
R², R³, R⁴ et R⁵ représentent, indépendamment les uns des autres, un atome d'hydrogène ou d'halogène, un groupe nitro, alkyle en C₁-C₄, alkyloxy en C₁-C₉, trifluorométhyle, trifluorométhoxy, ou des groupes aryl(C₆-C₁₀)-alkyloxy(C₁-C₄), aryloxy en C₆-C₁₀, aryle en C₆-C₁₀, cycloalkyle en C₃-C₈ ou O-cycloalkyle(C₃-C₈) qui peuvent être une, deux ou trois fois substitués par halogène, CF₃, alkyloxy en C₁-C₄ ou alkyle en C₁-C₄ ;
ainsi que leurs sels et sels d'addition avec des acides pharmacologiquement acceptables.

2. Composés de formule 1 selon la revendication 1, dans lesquels
R² représente un atome d'hydrogène ou d'halogène, un groupe alkyle en C₁-C₄ ou alkyloxy en C₁-C₉.

3. Composés de formule 1 selon les revendications 1 et 2, dans lesquels
R³ représente un atome d'hydrogène, un groupe alkyle en C₁-C₄, trifluorométhoxy, aryl(C₆-C₁₀) - alkyloxy(C₁-C₄) qui peut éventuellement être substitué par halogène dans le fragment aryle.

4. Composés de formule 1 selon les revendications 1 à 3, dans lesquels
R⁴ représente un atome d'hydrogène, le groupe trifluorométhoxy ou chlorophénoxy.

5. Composés de formule 1 selon les revendications 1 à 4, dans lesquels
R⁵ représente un atome d'hydrogène.

6. Composés de formule 1 selon les revendications 1 à 5, dans lesquels
R¹ représente un groupe alkyle en C₈-C₁₆.

7. Composés de formule 1 selon les revendications 1 à 6, dans lesquels
R¹ représente un groupe alkyle en C₈-C₁₆,
R² représente un atome d'hydrogène,
R³ représente un atome d'hydrogène ou le groupe trifluorométhoxy,
R⁴ représente un atome d'hydrogène, le groupe trifluorométhoxy ou 4-chloro-phénoxy et
R⁵ représente un atome d'hydrogène.

8. Procédé pour la préparation des composés de formule 1 selon les revendications 1 à 7, **caractérisé par** 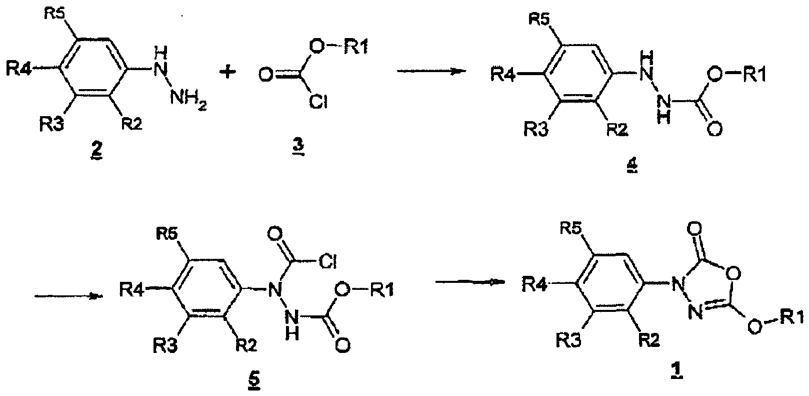 mise en réaction d'hydrazines de formule générale 2 avec des esters d'acide chloroformique de formule 3 ou d'autres dérivés réactifs d'esters d'acides carboniques, formules dans lesquelles R¹, R², R³, R⁴ et R⁵ sont tels que définis dans les revendications 1 à 7, conduisant aux composés de formule 4, qui sont acylés avec du phosgène, du carbonyldiimidazole, du diphosgène ou du triphosgène, cyclisés et éventuellement convertis en les composés de formule 1 par une nouvelle modification chimique des radicaux R²-R⁵.

9. Médicament contenant au moins un composé de formule 1 selon les revendications 1 à 7.

10. Médicament destiné au traitement de l'obésité, contenant au moins un composé de formule 1 selon les revendications 1 à 7.

11. Utilisation d'au moins un des composés de formule 1 selon les revendications 1 à 7, pour la fabrication d'un médicament.
